# EUROPEAN PATENT APPLICATION

(11) **EP 1 033 406 A1**
(43) Date of publication of application: **06.09.2000**
(21) Application number: 98947801.1
(22) Date of filing: 09.10.1998
(51) Int. Cl.: C12N 15/62, C12P 21/08, C07K 16/28

(54) **INDUCER FOR PRODUCTION OF ANTIGEN-SPECIFIC ANTIBODY, EXPRESSION VECTOR CONTAINING GENE THEREFOR, AND METHOD OF INDUCING PRODUCTION OF ANTIGEN-SPECIFIC ANTIBODY**

(30) Priority: 27.10.1997 JP 29439397
(71) Applicant: Sumitomo Electric Industries, Ltd., Osaka-shi, Osaka 541-0041 (JP)
(72) Inventor: SAKAGUCHI, Nobuo, Kumamoto-shi Kumamoto 860-0085 (JP)
(74) Representative: Baldock, Sharon Claire
(86) International application number: JP9804563
(87) International publication number: WO9922008

(57) **Abstract**

The inducer for the production of an antigen-specific antibody according to this invention is a chimera molecule comprising an antigen and a substance providing a B cell with a signal that activates the B cell to produce an antigen-specific antibody with strong avidity to said antigen. The method for inducing the production of an antigen-specific antibody according to the invention is that which utilizes the aforementioned molecule as an inducer for the production of an antigen-specific antibody and simultaneously stimulates an antigen receptor of a B cell and a receptor that activates the B cell to produce the antigen-specific antibody.

## Description

### TECHNICAL FIELD

This invention relates to an inducer for the production of an antigen-specific antibody, an expression vector containing a gene therefor, and a method for inducing the production of the antigen-specific antibody.

### BACKGROUND ART

Humans may evade infections by a variety of pathogens, such as viruses and bacteria, and by malignant tumors, such as cancers, and can lead healthy lives: this results from exclusion of foreign matters or pathogens by their biological defense mechanisms. The biological defense mechanism is also referred to as "the immune system," and such immune system comprises two systems: natural immunity (or natural immune system) and acquired immunity (or acquired immune system).

The natural immune system is one responsible for the initial defense against foreign matters, pathogens and the like from the outside, and it excludes most pathogens before they have caused apparent infections. This natural immune system does not specifically react to a particular antigen (foreign matter or pathogen), but exhibits the same responsiveness to all antigens. The cells that function in the natural immune system are neutrophiles, monocytes, microphages, NK cells, etc. Humans defend themselves from infection by pathogens because these cells release substances such as lysozyme, complements, and acute phase reaction proteins (interferons, CRP).

Resistance by the natural immune system can not be enhanced even if the same object (foreign matter or pathogen) has repeatedly infected the system.

The acquired immune system, which is the other of the two systems, reacts specifically to each source of infection and normally acts to exclude the source of infection. That is, in the acquired immune system the cells, which are referred to as "lymphocytes," remove the source of infection outside the living body by killing the infected cells or neutralizing the foreign matters with antibodies. Among lymphocytes, T cells (T lymphocytes) act to kill the infected cells (function as killer cells) and help B cells (B lymphocytes) that produce antibodies (function as helper cells). When the source of infection enters into the body from the outside, B cells act to produce an antibody that most efficiently reacts with the source of infection by undergoing mutation repeatedly.

One of the characteristics of the acquired immune system is enhancement in resistance as a result of repeated infection by the same object (foreign matter or pathogen). Examples include infectious diseases, such as measles and mumps, that youngsters catch. They will never suffer from the same diseases once infected with these in their infancy.

This phenomenon is generally referred to as "acquiring immunity against the disease." It is antibodies that contribute to the leading role in the acquired immune system. An antibody is a protein that B cells produce. Such a protein (antibody) is characterized in that it reacts with an antigen on a one-to-one basis: the antigen means what is different from the substances inherent in the living body, the examples of which include invasive foreign matters, pathogens, and cancer cells that appear through mutation in the living body. There are many substances that can possibly be antigens, and they are said to be approximately over one billion. Thus the same number of antibodies will be produced which react with the antigens.

Furthermore, such antibodies change into different types of antibodies possessing stronger avidity (stronger affinity) to antigens by being exposed to the antigens many times. This mechanism is a characteristic that the immune cells have; B cells cause genes of antibodies to mutate so that the antibodies can bind to antigens through stronger affinity. The process, however, often last as long as a few weeks. Thus, it will not be able to adequately cope with the situation where the host has been infected with a disease that has an acute and lethal symptom. This will necessitate a vaccine therapy by which the body is immunized by being administered with an antigen that has been non-toxicated or attenuated in advance, as well as a serum therapy where an antibody against the antigen has previously been prepared. On the other hand, the antibody that has been produced in the body is memorized by B cells: that is, the gene producing the antibody is preserved intact. If the host has been infected with the same antigen several times, the antibody will be quickly produced based on the memory. Therefore, it will prevent the same disease from developing a serious symptom.

### DISCLOSURE OF INVENTION

The immune system explained above, which is a biological defense, alone can, in principal, cope with infections (such as pest, smallpox, AIDS and Ebola hemorragic fever) and malignant tumors such as cancer. However, when the aforementioned immune system alone is directed to these infections, cancers or the like, it suffers a problem that a vast amount of time is needed before antigen-specific antibodies and T cells are produced in the body and an effective immune system is acquired. For example, the problem with the sources of infection (e.g., certain outermembrane proteins of AIDS) which themselves undergo many mutations is that acquisition of the above-mentioned immune system does not quite catch up with them.

It is said that in viral diseases, such as AIDS or Ebola hemorragic fever, about one out of 100,000 escapes from death and resumes a healthy body. This is thought to be ascribable to any of the following in the immune system of the particular individual: (1) the function of macrophages is powerful enough to produce a markedly large amount of substances such as cytokines that are induced during inflammation; (2) the viral antigen matches MHC class I or class II molecules of the individual and cytotoxic T cells may be sufficiently induced; and (3) the viral antigen matches MHC class I or class II molecules of the individual and B cells are sufficiently induced such that they produce antigen-specific antibodies. Here, the "antigen-specific" means strong avidity (not less than 10⁶ M⁻¹) to the antigen.

An object of this invention is to provide a method for inducing the production of such an antigen-specific antibody and an inducer for the production of the antigen-specific antibody which allows for its induction.

Specifically, the present inventors have diligently pursued further research with the aim toward attaining the object described above; and they have discovered a method for inducing the production of an antigen-specific antibody as described in (3) above and have successfully discovered a substance that enables the method. This invention has thus been accomplished. That is, the invention provides an inducer for the production of an antigen-specific antibody, comprising an antigen portion that is recognized by an antigen receptor of a B cell and a second signal transduction portion capable of providing the B cell with a second signal that the B cell requires to produce an antigen-specific antibody possessing strong avidity to the antigen.

The invention also provides the inducer for the production of an antigen-specific antibody described above, wherein the second signal transduction portion is an antibody against a CD40 molecule or an active fragment thereof that can recognize the CD40 molecule.

The invention also provides an expression vector containing a gene encoding the inducer for the production of an antigen-specific antibody.

Further, the invention provides a method for inducing the production of an antigen-specific antibody which comprises:
carrying out the immunization with an inducer for the production of an antigen-specific antibody, said inducer comprising an antigen portion that is recognized by an antigen receptor of a B cell and a second signal transduction portion capable of providing the B cell with a second signal that the B cell requires to produce an antigen-specific antibody possessing strong avidity to the antigen;
thereby providing the B cell with the second signal that the B cell requires to produce the antigen-specific antibody possessing strong avidity to the antigen simultaneously when the antigen receptor of the B cell binds to the antigen; and
causing the B cell to produce the antigen-specific antibody possessing a high affinity to the antigen.

The invention also provides the method for inducing the production of an antigen-specific antibody as described above, wherein the second signal is a signal of a CD40 molecule.

The method and substance according to the invention will be explained in detail by way of embodiments hereinbelow.

### BRIEF DESCRIPTION OF DRAWINGS

Fig. 1 is a graph showing a vector into which V_{H}-V_{L} has been inserted.
Fig.2 is a graph showing a vector into which psig-(V_{H}-V_{L}) and HIV-V3 fragments have been inserted.
Fig. 3 is a schematic representation of an anti-CD40(V_{H}-V_{L}) -antigen chimera molecule.
Fig. 4 is a graph showing a pBKS-H(#18) vector into which a gene encoding the V_{H}-CH1 portion of an anti-CD40 antibody that hybridoma LB429 produces.
Fig. 5 is a graph showing a pBKS-L(#4) vector into which a gene encoding the V_{H}-C_{L} portion of the anti-CD40 antibody that hybridoma LB429 produces.

### BEST MODE FOR CARRYING OUT THE INVENTION

As explained above, while this invention provides a method for inducing the production of an antigen-specific antibody and a substance therefor, the following applications will be enabled by using the method and the substance according to the invention.

Specifically, the invention will permit the development of a vaccine containing as an effective ingredient, the inducer for the production of an antigen-specific antibody according to the invention.

Also, the inducer for the production of an antigen-specific antibody according to the invention may include the one characterized in that the antigen portion of the inducer for the production of an antigen-specific antibody as described above is an outermembrane protein of human immunodeficiency virus.

Further, the invention will permit the development of the vaccine described above wherein the antigen portion of the inducer for the production of an antigen-specific antibody is an outermembrane protein of human immunodeficiency virus.

Still further, the invention will permit the development of a method capable of producing a monoclonal antibody by utilizing the inducer for the production of an antigen-specific antibody according to the invention.

Furthermore, the inducer for the production of an antigen-specific antibody according to the invention may include one in which the second signal transduction portion is a binding site to a CD40 molecule.

### Mechanism for antibody production and method for inducing production of antigen-specific antibody

The findings that the present inventors have gained on the mechanism of production of antibodies will be summarized in what follows.

IgM molecules, which bind to antigens on a one-to-one basis, serve as the receptor of B cells that are precursor cells producing antibodies, and the B cells are expressed on their surfaces. The antigen, exogenous or endogenous, ensures the production of an antibody by binding to the receptor on a one-to-one basis. In normal immune reactions, antigens and cytokinins derived from T cells first promote the proliferation of clones comprising only antigen-specific B cells. However, this alone produces only antibodies of IgM class in many cases and no antibodies possessing effective affinities from the standpoint of biological defense: this is referred to as "primary immune response."

When further exposed to antigens or subjected to booster, these antigen-specific B cells will be differentiated into cells producing antibodies of IgG class that have stronger avidity to the antigens. During this booster or the process of additional antigen exposure, it is required that a co-stimulatory signal besides the antigen, which enhances the affinity of the antibody, be transmitted to the antigen-specific B cells.

Among such co-stimulatory signals (or second signals) a signal of CD40 molecules is important. In other words, the CD40 molecules on the surfaces of B cells are important as the receptor of the co-stimulatory signal. The signal that is given CD40 molecules is usually generated by CD40L molecules that are present on activated T cells which serve as a ligand. In addition, it has been confirmed in human and murine in vitro systems or murine in vivo systems that such CD40L molecules can be substituted by antibodies against the CD40 molecules.

The present inventors have researched on substances that effectively progress the booster or the process of additional antigen exposure described above, and have discovered that as an inducer for the production of an antigen-specific antibody, a substance comprising an antigen portion and a second signal transduction portion that provides B cells with the co-stimulatory signal is ideal. It has been also found that an antibody against CD40 molecule or a fragment thereof which is an active fragment having a region requisite for reaction with CD40 molecules can, for example, be used as the second signal transduction portion.

Specifically, a complex between the antigen and the antibody against CD40 molecules or between the antigen and the active fragment of antibody having a region requisite for reaction with the CD40 molecules binds to a B cell on the same, single, B cell surface and then provides the B cell with the second signal, thereby inducing the production of a high affinity antibody. Furthermore, it has been found that the capability of substantially simultaneous binding to the B cell and of providing the B cell with the second signal is critical for the reasons described below. Where the binding between an antigen and IgM which is a receptor of the antigen is weak, B cells that express the IgM can not survive for a long period. In addition, although the signals from CD40 molecules enter to all of the B cells, the cells die in lymph nodes through apoptosis when the signal from the antigen is not simultaneous therewith. Further, even if the individual molecules are administered concurrently, it seldom happens, in reality, that the same B cell is simultaneously provided with stimulation from both, in vivo, because of problems such as circulation, half-life times and affinities. The formation of the complex described above will allow the same cell to be substantially subjected to simultaneous stimulation.

Based on the obtained findings described above it is evident that stimulation to B cells from the complex between the antigen and the antibody against CD40 molecules or between the antigen and the active fragment of antibody having a region requisite for reaction with the CD40 molecules is indispensable for inducing the production of effective antigen-specific antibodies by the B cells.

### Inducer for the production of antigen-specific antibody

Explanations will be made more concretely hereinbelow to the constitution of the inducer for the production of an antigen-specific antibody according to this invention.

The inducer for the production of an antigen-specific antibody according to the invention is a molecule having the constitution designed such that the antigen receptor on the surfaces of B cells and the receptor of the second signal which is co-stimulatory may be simultaneously stimulated as explained above: the molecule may be sometimes referred to as "chimera molecule." The method for inducing the production of an antigen-specific antibody according to the invention comprises preparing the chimera molecule, activating with the molecule, an individual B cell on a surface thereof substantially in a simultaneous manner, and inducing the production of an antigen-specific antibody.

For example, this can be made possible by converting a variable region (V region) of mouse anti-CD40 antibody into a single peptide, linking it with a Fc region, and further conjugating them to a viral antigen. The resulting chimera molecule can be produced as a solubilized molecule using COS cells and can readily be purified.

Mice are immunized with the chimera molecule. Thereby, the viral antigen portion of the chimera molecule is recognized by the antigen receptor of B cells in the mouse bodies; in a substantially simultaneous manner, the V region portion of the mouse CD40 antibody within the chimera molecule binds to the CD40 molecules expressed on the surfaces of B cells. This will realize that antigen-specific B cells are provided with two kinds of stimulation in a substantially simultaneous manner.

That is, where the antigen receptor of B cells possesses a low affinity to a viral antigen, the B cells can not survive for a long period. The cells also may die in lymph nodes through apoptosis. Therefore, only the B cell clones that express antigen receptors possessing strong avidity to the antigen will selectively be activated, allowing the production of antigen-specific antibodies to be induced.

There is no particular limitation to the experimental model, but mice may preferably be used. This is because effective anti-virus antibodies obtained with mice can be humanized in a relatively easy manner and their utility as the experimental model can be combined with the potential utility of their applications in antibody therapies.

### Preparation of anti-CD40 antibody-viral antigen chimera molecule

1. Antibodies from rats or hamsters can be used as antibodies against murine CD40 molecules for the purpose of humanizing the antibodies; particularly, those from rats are preferred because immunoglobulin from hamsters contain a large number of unknown members. It is therefore preferred that rat anti-mouse CD40 monoclonal antibody producing hybridomas be used. Hybridomas may be prepared by the so-called cell fusion technique. For example, a rat is immunized with mouse CD40, and spleen cells of the rat are fused with myeloma cells in the presence of polyethylene glycol to prepare the rat anti-mouse CD40 monoclonal antibody producing hybridoma. In this case, the antibody produced by the hybridoma is, preferably, one with strong capability to activate B cells. In administering to humans, the anti-CD antibodies are preferably humanized. Particularly, it is preferred that anti-human CD40 antibodies be humanized. The resulting anti-CD4O antibody-viral antigen chimera molecules can be used as vaccines for a variety of diseases (antigens).
2. Production of single peptide anti-CD40 V_{H}-V_{L} through genetic engineering can be made possible, for example, by preparing cDNA from the hybridoma described above, isolating genes for V_{H} and V_{L}, determining their sequences through DNA sequencing, and then incorporating them into a vector pslg for the preparation of a single peptide antibody.

This vector has a Fc portion of human immunoglobulin, produces a solublized anti-mouse CD40-human Fc protein by introducing genes into COS cells through gene transfer, and secretes the protein extracellularly. Thus SP2/O murine myeloma cells are, for example, used for culturing in a serum-free medium, and it will be possible to produce a large quantity of protein. For purification of the proteins to be obtained, a standard protein A column may preferably be used. If the human Fc portion poses a problem as the immunogen, the protein may be purified with a nickel column by attaching Tag to the protein which facilitates purification. When this method is to be employed, the human Fc portion should preferably be eliminated from the vector.

### Confirmation of induction of the production of antigen-specific antibody by anti-CD40 antibody-viral antigen chimera molecule

After the construction of the chimera molecule has been confirmed, its effectiveness as an antigen can be tested, for example, in an in vitro culture system. Specifically, the possibility of B cell stimulation, the inducibility of production of antibodies against viral antigens, and the specificity of the antigens to which the antibodies bind (i.e., affinities for antigens) can be tested in vitro. Furthermore, it is also possible to determine whether or not the avidity of an anti-viral antibody to bind to an antigen increases, namely whether or not the production of an antigen-specific antibody is induced by directly injecting a mouse with the molecule as the antigen. If necessary, for the purpose of preparing antibodies with higher affinities or antibodies with high neutralizing activities, the quantities of antigen, methods of injection, methods of injection in combination with adjuvants, methods of intraperitoneal injection, footpad subcutaneous injection, and intravenous injection and the like may be examined.

### Concrete examples of induction of the production of antigen-specific antibody

The concrete method described below can, for example, be used to confirm induction of the production of an antigen-specific antibody by a chimera molecule.

### 1. Immunization

Chimera molecule (1 ml, 1 mg) and Freund complete adjuvant (1 ml) are mixed to prepare an emulsion. Three Balb/c mice, 6-weeks of age, are immunized with the resulting mixed emulsion on their footpads. It is preferred to use as a control, an adjuvant with which only 19 amino acids of an antigen are mixed or an adjuvant with which 19 amino acids of the antigen and an anti-CD40 antibody are mixed. Approximately one week later, the adjuvant is switched to an incomplete one, and it is administered to the mice intraperitoneally. Further, after immunization four times at intervals of one week, blood (100 µl each) is collected from each mouse. Antibody titers are determined by ELISA.

### 2. ELISA

Measurement can be made according to the following protocol.
(I) An antigen solution (preferably containing a 19-amino acid peptide) is diluted with PBS to 25 µg/ml. The solution is fractionally dispensed to each well of a 96-well ELISA plate (e.g., Xenobind from Xenopore) at 50 µl per well. The solution is then allowed to stand at 4 ° C overnight.
(II) The antigen solution is discarded. Blockace (Dainippon Pharmaceutical Co. Ltd.) diluted 4-fold with distilled water is fractionally dispensed to each well at 200 µg per well. Blocking is carried out by allowing the plate to stand at room temperature for 2 h.
(III) The blocking solution is discarded. Antiserum is added to the plate as a primary antibody to be tested. The antiserum has been serially diluted with PBS and 20-, 40-, and 80-fold dilutions and so on, in this order, are fractionally dispensed to 96 wells at 50 µl per well from the first column up to the 12th column. Thus, the 12th column will have a concentration of one 40960th of the stock serum. After dispensing, the plate is allowed to stand at room temperature for about one hour.
(IV) The primary antibody solutions are discarded, and the plate is washed with 0.05% Tween20/PBS. Subsequently, biotinylated anti-mouse IgG antibody diluted with PBS 1000-fold, which serves as a secondary antibody solution, is fractionally dispensed to each well at 50 µl per well. The plate is then allowed to stand at room temperature for 30 min.
(V) After discarding the secondary antibody solution, the plate is washed with 0.05% Tween/PBS. ABC solution, such as one available from Vector, diluted with PBS 1000-fold is fractionally dispensed to each well at 50 µl per well. The plate is then allowed to stand at room temperature for 30 min.
(VI) After discarding the ABC solution, the plate is washed with 0.05% Tween/PBS. OPD (orthophenylenediamine)-H₂O₂/PBS is fractionally dispensed to each well at 100 µl per well. After development of sufficient color, the reaction is quenched with 2N sulfuric acid, and absorbance at 490 nm is measured using a microplate reader.

When the animal immunized with the chimera molecule is found to exhibit antibody titers substantially greater than those of the control group, it will ascertain the induction of production of an antigen-specific antibody according to the invention.

### Model for preparation of chimera molecules

For the antigen portion of a chimera molecule, HIVq120 may preferably be employed, for example. For a viral epitope, a consensus sequence of 19 amino acids in the V3 loop of gp120 may preferably be employed. That is, such portion is believed to be the portion that binds to CC-CKR-5 which acts as a co-receptor when a virus is incorporated into a cell. It is thus known to be the epitope capable of producing an antibody with the highest neutralizing activity.

Mice are immunized with the chimera molecule, and lymphoma cells and myeloma cells are fused to establish hybridomas producing anti-gp120 monoclonal antibody in as many numbers as possible which possess high neutralizing activity. mRNA is purified from the monoclonal antibody producing hybridoma, cDNA is synthesized, and immunoglobulin genes may be obtained.

It is also possible to directly use mouse monoclonal antibodies, though they may preferably be converted into humanized monoclonal antibodies through gene manipulation techniques. Concurrently, single peptide antibodies can be prepared using a psIg vector. A large number of monoclonal antibodies against the gp120 protein have been produced around the world; however, there have hitherto been no antibodies with high affinity, nor there have been any antibodies with high neutralizing activity.

In the present specification and drawings, where bases and amino acids are represented by abbreviations, the abbreviations according to IUPAC-IUB or those employed conventionally in the art are used: "X" further represents an arbitrary amino acid.

### EXAMPLES

This invention will be explained in greater detail by way of the following examples hereinbelow; however, the invention is not to be limited to these examples.

A chimera molecule between an anti-CD40 antibody and an antigen was prepared in the manner described below.

Preparation of the anti-CD40 antibody was accomplished using hybridoma LB429 (which was established at the Department of Immunology, School of Medicine, Kumamoto University). The antibody was rat anti-mouse CD40 antibody and possessed strong B cell activation capability.

### (1) Preparation of Single Peptide Anti-CD40 V_{H} Linker-V_{L} Single Fragment

### (1)-1 Preparation of cDNA

cDNA was prepared from the hybridoma described above. Hybridoma LB429 cells were cultured in a 25-cm² flask. The cultured cells were harvested and washed with PBS under centrifugation, after which the cells were suspended in 1 ml of PBS and the cell number was counted. Cells (1x10⁶) were taken into an aseptic Eppendorf tube, the supernatant was removed through centrifugation, and the pellet was subjected to tapping.

RNAzolB (Cosmo Bio Co. Ltd.), 200 µl, was added to the pellet and cells were dissolved by stirring thoroughly with the tip of a PIPETTEMAN (registered trademark, Gilson Inc.). After 20 µl of chloroform was added and shaken, the cells were allowed to stand in water for 5 min. Centrifugation at 15,000 rpm for 15 mm at 4 °C was carried out and a colorless portion of the upper layer was then collected and transferred to a new tube. After centrifugation at 15,000 rpm for 15 min at 4 °C, the supernatant was discarded and 800 µl of 75% ethanol was added to the pellet, which was allowed to stand at -20 °C for 30 min. Centrifugation was carried out at 15,000 rpm for 15 min at 4 °C and 11.5 µl of distilled water was then added to the pellet.

Oligo-dT (0.5 mg/ml, 0.5 µl) was added to the solution and it was allowed to stand at 70 °C for 10 min and on ice for 5 min. To this was added a mixture having the composition described below and it was allowed to stand at 42 °C for 50 min, at 90 °C for 5 min, and on ice for 5 min.

| | |
|---|---|
| 5xRT Buffer | 4 µl |
| 10 mM dNTPmix | 1 µl |
| Superscript Rtase (Stratagene Inc.) | 1 µl |

RnaseH (1 µl) was added to the above mixture and it was allowed to stand at 37 °C for 20 min to prepare a cDNA mixture.

To 20 µl of the single-stranded cDNA solution described above was added a mixture having the composition described below and it was allowed to stand at 12 °C for 30 min and at 22 °C for 1 h.

| | |
|---|---|
| 4xpol Buffer | 25 µl |
| dNTP | 5 µl |
| *E. coli* DNA polymerase | 3 µl |
| *E. coli* DNA ligase | 1 µl |
| DDW | 66 µl |

To this reaction solution were added 50 µl of phenol and 50 µl of chloroform, and after shaking, centrifugation was carried out. The supernatant was charged onto a SizeSep-400 Spun Column (Amersham Pharmacia Biotech) and it was centrifuged at 1200 rpm for 2 min to prepare 100 µl of double-stranded cDNA.

To 30 µl of the resulting double-stranded cDNA was then added a mixture having the composition described below and it was allowed to stand at 16 °C for 2 h.

| | |
|---|---|
| Spermidine | 0.5 µl |
| DTT | 1 µl |
| Ligation buffer | 70 µl |
| PEG | 30 µl |
| ATP | 1 µl |
| T4 DNA ligase | 1 µl |
| Adaptor | 2 µl |

Here, a Marathon cDNA Amplification Kit (Clontech Laboratories, Inc.) was used as an adapter. The resulting reaction solution was extracted with phenol/chloroform, and then a Spun Column was used to prepare a double-stranded cDNA with the adapter attached thereto.

### (1)-2 PCR reaction

The cDNA obtained above was used to perform PCR under the following conditions.

AP1 (CCATCCTAATACGACTCACTATAGGGC as set forth in SEQ ID NO: 5 in the Sequence Listing; Clontech Laboratories, Inc.) and 3VH (GTGGATAGACAGATGGGGTCTGTTG as set forth in SEQ ID NO: 6 in the Sequence Listing) were used as the V_{H} primer, and AP1 and 3VL (GGATGCATTGGTGCAGCATCAGCC as set forth in SEQ ID NO: 7 in the Sequence Listing) as the V_{L} primer.

| | V_{H} | V_{L} |
|---|---|---|
| CDNA | 2 µl | 2 µl |
| DNTPmix | 1 µl | 1 µl |
| Primer (1mM) | 2 µl | 1 µl |
| 10xPCR buffer | 4 µl | 4 µl |
| DDW | 30.5 µl | 31.5 µl |
| Taq polymerase (Ampli-Taq) | 0.5 µl | 0.5 µl |

Mineral oil (400 µl) was overlaid onto this solution, and it was allowed to stand at 94 ° C for 5 min. Subsequently, the following cycle was repeated 30 times for reaction: at 55 ° C for 2 min, followed by at 720 ° C for 1 min, and followed by at 94 ° C for 1 min. The solution was then allowed to stand at 55°C for 2 mm, and at 72 ° C for 10 min.

After reaction, 4 µl of the PCR product was subjected to mini gel electrophoresis (1.5% agarose gel), which ascertained the amplification of V_{H} (H chain V region) and V_{L} (L chain V region) fragments.

### (1)-3 Recovery of V_{H} and V_{L} fragments

All the PCR products that had been prepared as described above was subjected to the mini gel electrophoresis to cut bands corresponding to the V_{H} and V_{L} fragments out of the gel. The PCR products were recovered from the cut gels by means of a Gene Clean kit (BIO 101 Inc.), and the recovered bands were reconfirmed by the mini gel electrophoresis.

### (1)-4 Ligation

A TA cloning kit (described below) was used to carry out DNA ligation under the following conditions.

| | |
|---|---|
| ADDW | 5 µl |
| 10xLigation buffer | 1 µl |
| PCR vector | 2 µl |
| PCR product | 1 µl |
| T4DNA Ligase | 1 µl |

Ligation was completed by allowing the reaction to continue at 14 °C overnight.

### (1)-5 Transformation

TA cloning kit was used to carry out transformation. To cells (competent *E. coli* cells attached to the kit), 50 µl, on ice were added 2 µl of 0.5 M 2-mercaptoethanol and the ligation mixture prepared in the foregoing section. After being allowed to stand for 30 min, the cells were further allowed to stand in a hot water bath at 42 °C for 30 sec and then, on ice for 20 min. A SOC medium (450 µl) was added to the cells, and the mixture was incubated at 225 rpm for 1 h at 37 °C.

Next, the incubated medium was spread onto a LB agar plate (+Amp, X-gal, and IPTG). Samples were made up at 50 µl, 100 µl, and 200 µl, respectively. After incubation at 37 °C for 18 h, the incubated mixture was allowed to stand at 4 °C for 2 h, when colonies in white and in blue appeared on the plate.

### (1)-6 Culturing and preparation on miniscale

For each white colony in the aforementioned sample, culturing was carried out in a 3-ml LB medium (+Amp). Thus 12 colonies were cultured and shaken at 37 °C overnight.

A cultured preparation (1.5 ml) for this cultured sample was taken into an Eppendorf tube: the remaining preparation was spread on a LB plate (+Amp), and cultured at 37 °C for preservation. The portion in the tube was centrifuged at 6,000 rpm for 2 min.

To the pellet was added 100 µl of Solution 1 (lysozyme 5 mg/ml), and the solution was allowed to stand at room temperature for 5 min, to which 200 µl of Solution 2 was then added. After addition of Solution 2, the mixture was gently stirred on ice and allowed to stand for 5 min. Subsequently, 150 µl of Solution 3 was added to the mixture and the mixture was allowed to stand on well-stirred ice for additional 15 min. Here, Solutions 1-3 employed the compositions that was described in the Manual-"Molecular Cloning 2nd Edition," Cold Spring Harbor Laboratory, 1989, p.1.25-1.28. Centrifugation was next carried out at 12,000 rpm for 5 min at 4 °C.

The supernatant was taken into a new Eppendorf tube. To this was added an equal volume of phenol saturated with water. Centrifugation was then carried out at 12,000 rpm for 1 min at room temperature.

The supernatant was taken into a new Eppendorf tube. To this were added 1 µl of Mussel glycogen and 900 µl of ethanol, and the mixture was allowed to stand at -80 °C for 30 min. Centrifugation was then carried out at 15,000 rpm for 5 min at 4 °C.

Next, the precipitates were dried. To this were added 20 µl of TE and 1 µl of RNase A (5 mg/ml), and the mixture was allowed to stand at 65 °C for 20 min. A plasmid was thus obtained.

After this plasmid was treated with restriction enzyme under the conditions described below, it was subjected to electrophoresis on a mini gel to ascertain the inserted fragments.

| | |
|---|---|
| High buffer | 1 µl |
| EcoRI/SalI | 1 µl |
| DNA | 1 µl |
| ADDW | 7 µl |

After incubation at 37 °C for 1 h, the plasmid was electrophoresed on a 0.75% agarose gel.

### (1)-7 DNA sequencing

The plasmid DNA (1 µl) was taken up and diluted with 99 µl of TE buffer. The absorbance (A260, A280, or A320) of this solution was measured and DNA contents were calculated (A260 of 1.0=50 µg/ml). The DNA solution was diluted with TE such that its DNA was 1 µg/ml as determined from A260 values. DNA sequencing was conducted on this sample according to the dye-terminator method. A Model 377 autosequencer from ABI was employed for sequencing.

### (1)-8 Analysis of V regions

The amino acid sequences of V regions were determined by computer analysis based on the DNA sequencers thus obtained. The amino acid sequence of V_{H} is set forth in SEQ ID NO: 1 and the amino acid sequence of V_{L} is set forth in SEQ ID NO: 2.

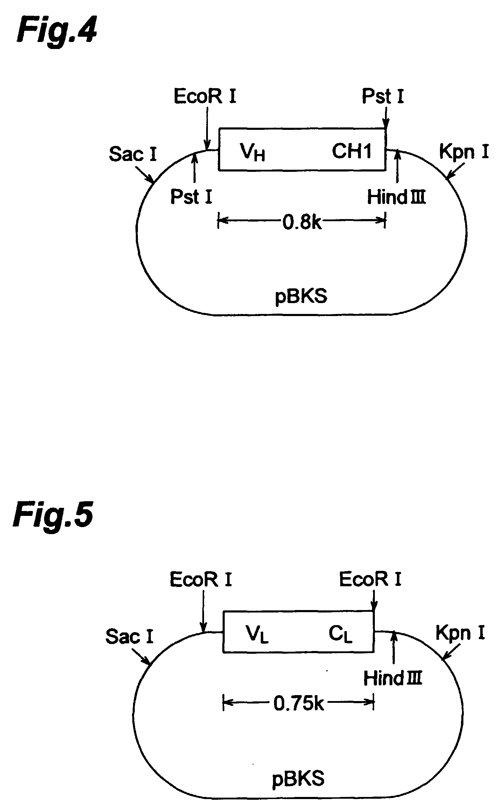

The gene encoding the V_{H}-CH1 portion of the anti-CD40 antibody that Hybridoma LB429 produced was inserted into a pBluescriptII(-) KS vector, and the vector was introduced into *E. coli* (DH10B) to prepare a transformant. In a similar manner, a transformant into which the gene encoding the V_{L}-C_{L} portion of the same antibody had been introduced was prepared. These transformants were designated pBKS-H (#18) and pBKS-L (#4), respectively. They were deposited on September 16, 1998 in the National Institute of Bioscience and Human-Technology, Agency of Industrial Science and Technology, Ministry of International Trade and Industry locating at 1-1-3 Higashi, Tsukuba, Ibaragi, Japan (Zip Code 305-8566) under the Budapest Treaty for international deposition. Their accession numbers were FERM BP-6504 and FERM BP-6503, respectively. The map for the vector having V_{H}-CH1 incorporated thereto is shown in Fig. 4 and the map for the vector having V_{L}-C_{L} incorporated thereto in Fig. 5.

### (1)-9 Preparation of V_{H}-linker-V_{L} single fragment

The V_{H} fragment and the V_{L} fragment were linked together using a linker primer available from Pharmacia. Specifically, the linker was attached between the V_{H} and V_{L} fragments under the following conditions: V_{H}-linker primer-CGATCCGCCACCGCCAGAGCCACCTCCGCCTGAACCGCCTCCACCTGA TGCTTCAGCTGAGGAGAC as set forth in SEQ ID NO: 8 and V_{L}-linker primer-TCAGGTGGAGGCGGTTCAGGCGGAGGTGGCTCTGGCGGTGGCGGATCG GGTGACACTGTACTGACC as set forth in SEQ ID NO: 9.

| | |
|---|---|
| V_{H} fragment | 2 µl |
| V_{L} fragment | 2 µl |
| dNTPmix | 1 µl |
| Linker primer | 2 µl |
| 10xPCR buffer | 4 µl |
| DDW | 28.5 µl |
| Tag polymerase (Ampli-Taq) | 0.5 µl |

Mineral oil (40 µl) was overlaid onto this solution, and it was allowed to stand at 94 °C for 5 min. Subsequently, the following cycle was repeated 7 times for reaction: at 55 °C for 2 min, followed by at 72 °C for 3 min, and followed by at 94 °C for 1 min. The solution was then allowed to stand at 55 °C for 2 min, and at 72 °C for 10 min.

After reaction, 4 µl of the PCR product was subjected to the mini gel electrophoresis (1.5% agarose gel), which ascertained the amplification of V_{H}-linker-V_{L} (abbreviated as V_{H}-V_{L} hereunder). Next, V_{H} (TAGGATCCTGAGGTACAGCTGGTGGAG as set forth in SEQ ID NO: 10) and V_{L} (CGGGATCCACTTACCTGTCCGTTTCAGTTCCAGCTT as set forth in SEQ ID NO: 11) were used to perform PCR according to (1)-2.

### (1)-10 Recovery of V_{H}-V_{L}

All the PCR products that had been prepared as described above were subjected to the mini gel electrophoresis to cut a band corresponding to V_{H}-V_{L} out of the gel. The PCR product was recovered from the cut gel by means of a Gene Clean kit (BIO 101, Inc.), and the recovered band was reconfirmed by the mini gel electrophoresis.

### (2) Linkage between V_{H}-V_{L} of the anti-CD40 Antibody and Fc-inserted Vector

### (2)-1 Preparation of V_{H}-V_{L}

First, V_{H}-V_{L} was cut with restriction enzyme BamHI. Specifically, after treatment with the restriction enzyme, V_{H}-V_{L} was subjected to the mini gel electrophoresis under the following conditions:

| | |
|---|---|
| B buffer (Boehringer Ingelheim GmbH) | 1 µl |
| BamHI | 1 µl |
| DNA | 1 µl |
| ADDW | 7 µl |

After incubation at 37 °C for 1 h, electrophoresis was carried on a 0.75% agarose gel.

### (2)-2 Ligation

Ligation was carried out between V_{H}-V_{L} and a human Fc-inserted vector psIg described below that had been cut with BamHI and treated with CIAP.

| | |
|---|---|
| ADDW | 5 µl |
| 10xLigation buffer | 1 µl |
| psIg | 2 µl |
| V_{H}-V_{L} | 1 µl |
| T4DNA Ligase | 1 µl |

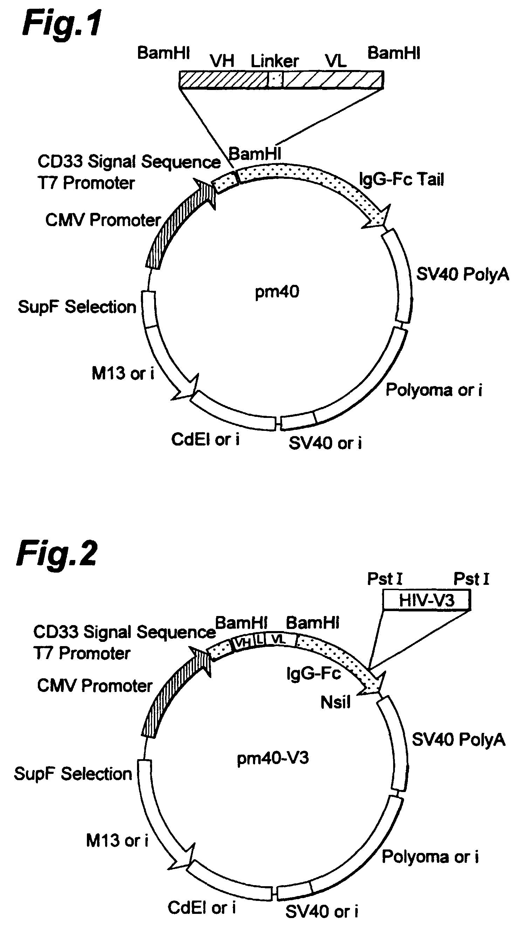

Ligation was completed by allowing the reaction to continue at 14 °C overnight (Fig. 1).

### (2)-3 Transformation

Similarly to (1)-5, transformation was carried out using *E. coli* MC1061/P3 strain.

### (2)-4 Culturing and preparation on miniscale

The same manipulations as those for (1)-6 were done on the colonies that appeared on a LB agar plate in (2)-3 to yield psIg-(V_{H}-V_{L}). Electrophoresis was, however, not carried out.

### (3) Linkage between Antigen Gene and (V_{H}-V_{L}-Fc) of the anti-CD4 Antibody

An antigen gene was linked to the resulting psIg-(V_{H}-V_{L}) in the following manner.

First, a sequence of V3 loop of gp120, which is an outermembrane protein of HIV, was to be used as an antigen for insertion. This sequence is a unique site at which there are few variations within gp120 with many variations. Therefore, from immunological surveys it is said that humans who have high antibody titers against this particular portion do not develop AIDS.

However, since the antibody titer against the portion would not increase according to conventional methods, the portion was considered to be a preferred example of the antigen for use to be targeted by this invention. Specifically, a partial gene of from No. 6971 to No. 7621 (SEQ ID NO: 4 in the Sequence Listing: the amino acid sequence encoded by the gene is set forth in SEQ ID NO: 3) that encodes V3 loop of HIV NL4-3 strain (Gen Bank Accession NO: M19921) was inserted into the downstream of human Fc of psIg-(V_{H}-V_{L}).

### (3)-1 Preparation of HIV-V3 fragment

With NL4-3 strain of T-tropic HIV as a template, 5'V3 primer (CTCTGCAGCCAGTAGTATCAACTCAACTG as set forth in SEQ ID NO: 12) and 3'V5 primer (CTCTGCAGTCTGAAGATCTCGGACCCATT as set forth in SEQ ID NO: 13) were used to perform PCR according to (1)-2.

The resulting PCR product was cut with restriction enzyme PstI and was electrophorezed on a 1% agarose gel. HIV-V3 fragment (667bp) was purified by means of a Gene Clean kit (BIO 101, Inc.).

### (3)-2 Ligation

psIg-(V_{H}-V_{L}) was treated with restriction enzyme NsiI. Specifically, treatment with the restriction enzyme was carried out under the following conditions:

| | |
|---|---|
| H buffer (Boebringer Ingelheim GmbH) | 1 µl |
| NsiI | 1 µl |
| DNA (1 µg) | 1 µl |
| ADDW | 6 µl |

After treatment at 37 °C for 1 h, the product was further treated with calf alkaline phosphatase. The conditions are as follows:

| | |
|---|---|
| Restriction enzyme-treated product | 10 µl |
| CIP (1 unit) | 1 µl |
| 10xCIP buffer | 2 µl |
| ADDW | 7 µl |

After treatment at 37 °C for 30 min, 2 µl of proteinaseK (20 mg/ml) was added to the solution. Further treatment at 37 °C for 30 min decomposed CIP. Subsequently, still further treatment at 65 °C for 30 min deactivated protainaseK. psIg-(V_{H}-V_{L}) that had thus been dephosphorylated was ligated to the HIV-V3 fragment.

| | |
|---|---|
| ADDW | 5 µl |
| 10xLigation buffer | 1 µl |
| psIg-(V_{H}-V_{L}) | 2 µl |
| HIV-V3 fragment | 1 µl |
| T4DNA ligase | 1 µl |

Ligation was completed by allowing the reaction to continue at 14 °C overnight.

### (3)-3 Transformation

Similarly to (1)-5, transformation was carried out using *E. coli* MN522 strain.

### (3)-4 Culturing and preparation on miniscale

The same manipulations as those for (1)-6 were done. No electrophoresis was, however, carried out. In this manner, psIg-(V_{H}-V_{L})-antibody gene was obtained.

### (4) Preparation of Chimera Molecule

### (4)-1 Preparation of anti-CD40 antibody (V_{H}-V_{L})-antigene chimera molecule

The psgIg-(V_{H}-V_{L})-antigene gene that had been obtained in (3) was introduced into myloma SP2/O. For gene transfer, the electroporation method was used with 1x10⁶ cells of SP2/O. Specifically, electroportion was carried out at 296V and 960 µF using a gene pulsar (Bio-Rad Laboratories Inc.). Cells were suspended into a 10%FCS/RPMI 1640 medium (5 ml). The cell suspension was dispensed to a 6-well plate and it was cultured. Then G418 (1 mg/ml) was added to the suspension as an antibiotic for selection. After culturing for 10 days, colonies were noted, and the cells were subjected to cloning by limiting dilution. Among the clones obtained, a clone was selected that had the highest expression rate of the introduced gene as determined by northern hybridization. Further, the clone was cultured. This served as an anti-CD 40 antibody (V_{H}-V_{L})-antigen chimera molecule producing cell.

### (4)-2 Production of chimera molecule

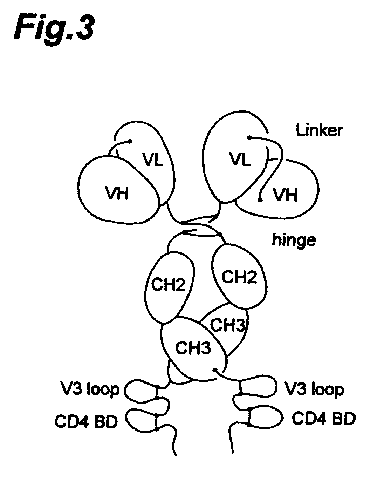

The anti-CD 40 antibody (V_{H}-V_{L})-antigen chimera molecule producing cells that had been prepared in the aforementioned process were cultured. Culturing employed an E-RDF serum-free medium (Kyokuto Pharmaceutical Co. Ltd.) and also a Technomouse (distributed by Gunze Sangyo Inc.) as a culturing system. Culturing for about one month yielded 20 mg of the chimera molecule. Purification was performed by directly passing the culture supernatant through a Protein A column (Amersham Pharmacia Biotech) following its manual. Fig. 3 shows a schematic representation of the chimera molecule.

### INDUSTRIAL APPLICABILITY

Use of the inducer for the production of an antigen-specific antibody according to this invention allows the time requisite for interaction between T cells and B cells to be shortened. Specifically, it obviates the need of time-consuming steps that ordinary immune systems involve: (I) antigen-presenting cells incorporate an antigen; (II) the antigen is presented against MHC; (III) T cells recognize MHC-antigen; (IV) the T cells, being activated, express CD40L on the surfaces thereof; and (V) the T cells interact with B cells.

Further even where presentation on MHC by antigens is difficult, the use of the inducer for the production of an antigen-specific antibody according to this invention allows for the interaction with B cells, thereby possibly finding applications in reagents for research purposes as well as in useful drugs such as vaccines.

Furthermore, the method for inducing the production of an antigen-specific antibody by the use of the inducer for the production of an antigen-specific antibody is useful for preparing novel monoclonal antibodies: utility as means for developing reagents for research purposes, dignostic agents and therapeutic agents.

Also, the expression vector for the inducer for the production of an antigen-specific antibody according to the invention is useful as a reagent for research purposes.

## Claims

1. An inducer for the production of an antigen-specific antibody, comprising an antigen portion that is recognized by an antigen receptor of a B cell and a second signal transduction portion capable of providing the B cell with a second signal that the B cell requires to produce an antigen-specific antibody possessing strong avidity to the antigen.

2. The inducer for the production of an antigen-specific antibody according to claim 1, wherein the second signal transduction portion is an antibody against a CD40 molecule or an active fragment thereof that can recognize the CD40 molecule.

3. An expression vector containing a gene encoding the inducer for the production of an antigen-specific antibody according to claim 1 or 2.

4. A method for inducing the production of an antigen-specific antibody which comprises:
carrying out the immunization with an inducer for the production of an antigen-specific antibody, said inducer comprising an antigen portion that is recognized by an antigen receptor of a B cell and a second signal transduction portion capable of providing the B cell with a second signal that the B cell requires to produce an antigen-specific antibody possessing strong avidity to the antigen;
thereby providing the B cell with the second signal that the B cell requires to produce the antigen-specific antibody possessing strong avidity to the antigen simultaneously when the antigen receptor of the B cell binds to the antigen; and
causing the B cell to produce the antigen-specific antibody possessing a high affinity to the antigen.

5. The method for inducing the production of an antigen-specific antibody according to claim 4, wherein the second signal is a signal of a CD40 molecule.


SEQUENCE LISTING

<110> SUMITOMO ELECTRIC INDUSTRIES, LTD

<120> Inducer for production of antigen-specific antibody, expression vector
containing gene therefor, and method for inducing production of
antigen-specific antibody

<130> SEI 98-30 PCT

<140> PCT/JP98/04563
<141> 1998-10-09

<150> JP 9/294393
<151> 1997-10-27

<160> 13

<210> 1
<211> 120
<212> PRT
<213> Rattus

<400> 1
Glu Val Gln Leu Val Glu Ser Gly Gly Gly Leu Val Gln Pro Gly Arg
 1               5                  10                  15
Ser Leu Lys Leu Ser Cys Ala Ala Ser Gly Phe Thr Phe Ser Asp Tyr
            20                  25                  30
Asn Met Ala Trp Val Arg Gln Ala Pro Lys Lys Gly Leu Glu Trp Val
        35                  40                  45
Ala Thr Ile Ser Tyr Asp Gly Ser Ser Thr Tyr Tyr Arg Asp Ser Val
    50                  55                  60
Lys Gly Arg Phe Thr Ile Ser Arg Asp Asn Ala Lys Ser Thr Leu Tyr
65                  70                  75                  80
Leu Gln Met Asp Ser Leu Arg Ser Glu Asp Thr Ala Thr Tyr Tyr Cys
                85                  90                  95
Ala Arg His Gly Gly Val Tyr Phe Asp Tyr Trp Gly Gln Gly Val Met
            100                 105                 110
Val Thr Val Ser Ser Ala Glu Ala
        115                 120

<210> 2
<211> 108
<212> PRT
<213> Rattus

<400> 2
Gly Asp Thr Val Leu Thr Gln Ser Pro Ala Leu Ala Val Ser Pro Gly
1               5                   10                  15
Glu Arg Val Thr Ile Ser Cys Arg Ala Ser Glu Ser Val Ser Thr Leu
            20                  25                  30
Met His Trp Tyr Gln Gln Lys Pro Gly Gln Gln Pro Lys Leu Leu Ile
        35                  40                  45
Tyr Leu Ala Ser Asn Leu Glu Ser Gly Val Pro Ala Arg Phe Ser Gly
    50                  55                  60
Ser Gly Ser Gly Thr Asp Phe Thr Leu Thr Ile Asp Pro Val Glu Ala
65                  70                  75                  80
Asp Asp Thr Ala Thr Tyr Tyr Cys Gln Gln Ser Trp Asn Asp Pro Tyr
                85                  90                  95
Thr Phe Gly Ala Gly Thr Lys Leu Glu Leu Lys Arg
            100                 105

<210> 3
<211> 217
<212> PRT
<213> Homo sapiens

<400> 3
Pro Val Val Ser Thr Gln Leu Leu Leu Asn Gly Ser Leu Ala Glu Glu
1               5                   10                  15
Asp Val Val Ile Arg Ser Ala Asn Phe Thr Asp Asn Ala Lys Thr Ile
            20                  25                  30
Ile Val Gln Leu Asn Thr Ser Val Glu Ile Asn Cys Thr Arg Pro Asn
        35                  40                  45
Asn Asn Thr Arg Lys Ser Ile Arg Ile Gln Arg Gly Pro Gly Arg Ala
    50                  55                  60
Phe Val Thr Ile Gly Lys Ile Gly Asn Met Arg Gln Ala His Cys Asn
65                  70                  75                  80
Ile Ser Arg Ala Lys Trp Asn Ala Thr Leu Lys Gln Ile Ala Ser Lys
                85                  90                  95
Leu Arg Glu Gln Phe Gly Asn Asn Lys Thr Ile Ile Phe Lys Gln Ser
            100                 105                 110
Ser Gly Gly Asp Pro Glu Ile Val Thr His Ser Phe Asn Cys Gly Gly
        115                 120                 125
Glu Phe Phe Tyr Cys Asn Ser Thr Gln Leu Phe Asn Ser Thr Trp Phe
    130                 135                 140
Asn Ser Thr Trp Ser Thr Glu Gly Ser Asn Asn Thr Glu Gly Ser Asp
145                 150                 155                 160
Thr Ile Thr Leu Pro Cys Arg Ile Lys Gln Phe Ile Asn Met Trp Gln
                165                 170                 175

Glu Val Gly Lys Ala Met Tyr Ala Pro Pro Ile Ser Gly Gln Ile Arg
            180                 185                 190
Cys Ser Ser Asn Ile Thr Gly Leu Leu Leu Thr Arg Asp Gly Gly Asn
        195                 200                 205
Asn Asn Asn Gly Ser Glu Ile Phe Arg
    210                 215

<210> 4
<211> 651
<212> DNA
<213> Homo sapiens

<221> CDS
<222> (1)..(651)

<400> 4
cca gta gta tca act caa ctg ctg tta aat ggc agt cta gca gaa gaa 48
Pro Val Val Ser Thr Gln Leu Leu Leu Asn Gly Ser Leu Ala Glu Glu
1               5                   10                  15

gat gta gta att aga tct gcc aat ttc aca gac aat gct aaa acc ata 96
Asp Val Val Ile Arg Ser Ala Asn Phe Thr Asp Asn Ala Lys Thr Ile
            20                  25                  30

ata gta cag ctg aac aca tct gta gaa att aat tgt aca aga ccc aac 144
Ile Val Gln Leu Asn Thr Ser Val Glu Ile Asn Cys Thr Arg Pro Asn
        35                  40                  45

aac aat aca aga aaa agt atc cgt atc cag agg gga cca ggg aga gca 192
Asn Asn Thr Arg Lys Ser Ile Arg Ile Gln Arg Gly Pro Gly Arg Ala
    50                  55                  60

ttt gtt aca ata gga aaa ata gga aat atg aga caa gca cat tgt aac 240
Phe Val Thr Ile Gly Lys Ile Gly Asn Met Arg Gln Ala His Cys Asn
65                  70                  75                  80

att agt aga gca aaa tgg aat gcc act tta aaa cag ata gct agc aaa 288
Ile Ser Arg Ala Lys Trp Asn Ala Thr Leu Lys Gln Ile Ala Ser Lys
                85                  90                  95

tta aga gaa caa ttt gga aat aat aaa aca ata atc ttt aag caa tcc 336
Leu Arg Glu Gln Phe Gly Asn Asn Lys Thr Ile Ile Phe Lys Gln Ser
            100                 105                 110

tca gga ggg gac cca gaa att gta acg cac agt ttt aat tgt gga ggg 384

Ser Gly Gly Asp Pro Glu Ile Val Thr His Ser Phe Asn Cys Gly Gly
        115                 120                 125

gaa ttt ttc tac tgt aat tca aca caa ctg ttt aat agt act tgg ttt 432
Glu Phe Phe Tyr Cys Asn Ser Thr Gln Leu Phe Asn Ser Thr Trp Phe
    130                 135                 140

aat agt act tgg agt act gaa ggg tca aat aac act gaa gga agt gac 480
Asn Ser Thr Trp Ser Thr Glu Gly Ser Asn Asn Thr Glu Gly Ser Asp
145                 150                 155                 160

aca atc aca ctc cca tgc aga ata aaa caa ttt ata aac atg tgg cag 528
Thr Ile Thr Leu Pro Cys Arg Ile Lys Gln Phe Ile Asn Met Trp Gln
                165                 170                 175

gaa gta gga aaa gca atg tat gcc cct ccc atc agt gga caa att aga 576
Glu Val Gly Lys Ala Met Tyr Ala Pro Pro Ile Ser Gly Gln Ile Arg
            180                 185                 190

tgt tca tca aat att act ggg ctg cta tta aca aga gat ggt ggt aat 624
Cys Ser Ser Asn Ile Thr Gly Leu Leu Leu Thr Arg Asp Gly Gly Asn
        195                 200                 205

aac aac aat ggg tcc gag atc ttc aga 651
Asn Asn Asn Gly Ser Glu Ile Phe Arg
    210                 215

<210> 5
<211> 27
<212> DNA
<213> Artificial Sequence

<220>
<223> Primer

<400> 5
ccatcctaat acgactcact atagggc 27

<210> 6
<211> 25
<212> DNA
<213> Artificial Sequence

<220>
<223> Primer

<400> 6
gtggatagac agatggggtc tgttg                                          25

<210> 7
<211> 24
<212> DNA
<213> Artificial Sequence
<220>
<223> Primer

<400> 7
ggatgcattg gtgcagcatc agcc                                           24

<210> 8
<211> 66
<212> DNA
<213> Artificial Sequence

<220>
<223> Primer

<400> 8
cgatccgcca ccgccagagc cacctccgcc tgaaccgcct ccacctgatg cttcagctga 60

ggagac                                                               66

<210> 9
<211> 66
<212> DNA
<213> Artificial Sequence

<220>
<223> Primer

<400> 9
tcaggtggag gcggttcagg cggaggtggc tctggcggtg gcggatcggg tgacactgta 60

ctgacc                                                               66

<210> 10
<211> 27
<212> DNA
<213> Artificial Sequence

<220>
<223> Primer

<400> 10
taggatcctg aggtacagct ggtggag                                          27

<210> 11
<211> 36
<212> DNA
<213> Artificial Sequence

<220>
<223> Primer

<400> 11
cgggatccac ttacctgtcc gtttcagttc cagctt                                36

<210> 12
<211> 29
<212> DNA
<213> Artificial Sequence

<220>
<223> Primer

<400> 12
ctctgcagcc agtagtatca actcaactg                                        29

<210> 13
<211> 29
<212> DNA
<213> Artificial Sequence

<220>
<223> Primer

<400> 13
ctctgcagtc tgaagatctc ggacccatt                                        29